Europäisches Patentamt

European Patent Office

Office européen des brevets

(19)

(11) Publication number: **0 068 344**
**A1**

# EUROPEAN PATENT APPLICATION

(12)

(21) Application number: 82105339.4

(22) Date of filing: 18.06.82

(51) Int. Cl.³: **G 01 N 33/54**

(30) Priority: **19.06.81 JP 93956/81**

(43) Date of publication of application: **05.01.83**
**Bulletin 83/1**

(84) Designated Contracting States: **BE CH DE FR GB IT LI NL SE**

(71) Applicant: **Eisai Co., Ltd., 6-10, Koishikawa 4-chome Bunkyo-ku, Tokyo 112 (JP)**

(72) Inventor: **Naraki, Tohru, 80-299, Akutamisuwayama Gifu-shi, Gifu Prefecture (JP)**
Inventor: **Sawada, Takashi, 2, Takehayamachi Kawashima-cho, Hashima-gun Gifu Prefecture (JP)**
Inventor: **Tsuiki, Nobuaki, 1-357, Suei-cho, Kagamigahara-shi Gifu Prefecture (JP)**
Inventor: **Tohda, Minoru, 2-23, Ryokuennaka, Kagamigahara-shi Gifu Prefecture (JP)**

(74) Representative: **Hansen, Bernd, Dr.rer.nat. et al, Hoffmann . Eitle & Partner Patentanwälte Arabellastrasse 4, D-8000 München 81 (DE)**

(54) Method and reagent kit for measurement using an enzyme-labelled antibody.

(57) A method for measuring the amount of an antigen or antibody in a serum by the solid phase method using an enzymelabelled antibody, which comprises adding before the measurement one reagent or a combination of two reagents selected from the group consisting of EDTA, kaolin, an antigen-antibody complex, an ammonium salt, zymosan and anti-Clq to the serum or the antigen-antibody reaction system involved in the measurement, and a reagent kit therefor. By use of such method and reagent kit, a non-specific measurement inhibitor present in the serum may be removed.

EP 0 068 344 A1

## METHOD AND REAGENT KIT FOR MEASUREMENT USING AN ENZYME-LABELLED ANTIBODY

The present invention relates to a method and reagent kit for measuring the amount of an antigen or antibody in a serum by the solid phase method using an enzyme-labelled antibody. More particularly, it relates to a method and reagent kit for measuring the amount of an antigen or antibody in a serum by the solid phase method using an enzyme-labelled antibody, which comprises, as one of the constitutional requirements, the removal of a non-specific inhibitor present in the serum.

On measuring the amount of an antigen or antibody in a serum, a technique to measure it by the solid phase method using an enzyme-labelled antibody has become widely employed as a safe and simple method.

However, it is observed that the values of an antigen or antibody obtained by measurement by said method extremely vary depending on the time expired between blood taking and measuring, and thus that a constant value is not obtained. One of the reason of this, for example, as reported in Clinica Chemica Acta, Vol. 87, 367 - 372 (1978), is due to the influences of a certain inhibitor present in a serum sample.

- 2 -

In other words, the inhibitor forms its own film along the solid phase surface and becomes a barrier between the antigen and the antibody, thereby blocking the reaction between them. Therefore, it can be seen that this blockade is a non-specific phenomenon which has nothing to do with the kind and amount of the antigen and antibody. Further, since the amount of the inhibitor in a serum is not always constant and varies depending on the time expired between blood taking and measuring, the conditions for pretreatment of a serum sample prior to measurement etc., the degree of inhibition varies widely and in some cases 100% inhibition could occur.

Under such circumstances, in order to accurately measure the amount of the antigen or antibody in the serum by the solid phase method using an enzyme-labelled antibody, it is necessary to eliminate the influences of the inhibitor in the serum. From such an aspect, we have been intensively searching for a means to remove the inhibitor or eliminate the inhibiting influences thereof and, as a result, have discovered that the aimed purpose can be achieved by previously adding one reagent or a combination of two reagents selected from the group consisting of EDTA, kaolin, an antigen-antibody complex, an ammonium salt, zymosan and anti-Clq to the serum or the antigen-antibody reaction system involved in the measurement, thus having accomplished the present invention.

- 3 -

Accordingly, the purpose of the present invention is to permit accurate measurement of the amount of an antigen or antibody in a serum by eliminating influences due to an inhibitor present in a serum sample by previously adding one reagent or a combination of two reagents selected from the group consisting of EDTA, kaolin, an antigen-antibody complex, an ammonium salt, zymosan and anti-Clq to the serum or the antigen-antibody reaction system involved in the measurement.

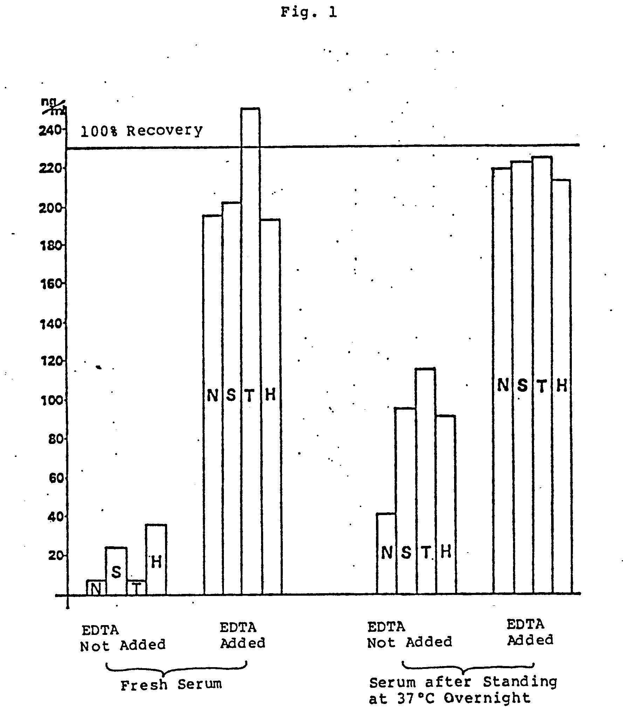

Fig. 1 shows the percent recovery of each measured value of $\alpha$-fetoprotein for various specimens.

The feature of the present invention is that the percent recovery of measurement is at such a high level as 90 – 100%, and therefore the amount of an antigen or antibody in a serum can be accurately measured.

The additive to be used in the present invention is one reagent or a combination of two selected reagents from the group consisting of EDTA, kaolin, an antigen-antibody complex, an ammonium salt, zymosan and anti-Clq. Among those, EDTA (ethylenediaminetetraacetic acid and salts thereof) is most preferred in view of the inhibition eliminating effect, simplicity of use and safety of the substance itself. On measuring, EDTA may be directly added to the serum or may be added to the antigen-antibody reaction system involved in the measurement. That is, it is possible to directly add EDTA to the serum to inactivate the inhibitor in the serum, or alternatively, it is possible to previously add a dilution containing EDTA to a measuring cup in which the antigen or antibody has been coated on its solid phase and then add a sample serum thereto to cause the antigen-antibody reaction involved in the measurement. Kaolin may be used only in the case where it is directly added to the serum, allowed to stand at room temperature, centrifuged to obtain a supernatant and the supernatant is measured. For example, kaolin may be added to a fresh serum to a concentration of 10 mg/ml, allowed to stand at room temperature for an hour, centrifuged at 3,000 rpm for 20 minutes, and the supernatant is thus recovered. The antigen-antibody complex also can be used only in the case where it is added to a serum, allowed to stand at room temperature, centrifuged to obtain a supernatant and the supernatant is measured. As the antigen-antibody complex, an immune complex obtained in the NHS (normal human serum) - anti-NHS reaction may be used. In this case, said complex is added to a fresh serum to a concentration of e.g. 2 mg/ml or so, allowed to stand at room temperature for 30 minutes, and centrifuged to obtain a supernatant for measurement. Zymosan and anti-Clq may be used in the case where they are either directly added to a serum, or added to the antigen-antibody reaction system involved in the measurement, without subjecting to centrifugation. Similarly, the ammonium salt may be used where it is directly added to a serum, which is then added to the reaction system without subjecting to centrifugation. As the ammonium salt, ammonium sulfate, for example, may be selected, while the amount of the additive to be added varies depending on the kind of the additive. In the case of EDTA, for example, the final concentration of 10 mM or higher may be satisfactory for exact measurement. In the case of ammonium sulfate, the final concentration of 200mM or higher may be satisfactory.

The present invention may be applied to any embodiment which involves the solid phase method using an enzyme-labelled antibody, and, for example, it is effective in methods included in the concept of the solid phase method using an enzyme-labelled antibody, such as a sandwich method, a competition method, a two-antibody method etc.

Further, as described hereinabove, since the inhibition phenomenon involved in the present invention is non-specific, the present invention is not restricted by the kinds of the antigen and antibody. For example, as shown in Test Example described hereinbelow, it is effective in any case where the antigen is $\alpha$-feto protein, m-aldolase or HBs antigen.

Furthermore, the inhibition phenomenon involved in the present invention is remarkable when measuring a fresh serum immediately after blood taking. This is presumed due to the inactivation of the inhibitor in the serum with time,

- 6 -

But, since the present invention is effective as long as the inhibitor is present in the serum, the present invention is not restricted by the time expired between blood taking and measuring.

The effect of the present invention is demonstrated by the follwoing Test Examples.

Test Example 1

Samples

Concentration of antigen obtained by adding EDTA to two fresh serum sepcimens taken from healthy humans F and K to the final concentrations for measurement in 6 steps described in Table 1.

Method

0.05 M Tris hydrochloric acid buffer (pH 8.0) containing 30 µg/ml of anti-m-aldolase antibody was added to a measuring cup, allowed to stand at 4°C overnight, then removed from the cup, which was then washed with water, a sample was added thereto and the reaction was incubated at 37°C for 60 minutes followed by washing. Thereafter, 100 µl of a solution obtained by diluting an alkali phosphatase-labelled anti-m-aldolase antibody with a normal rabbit serum to an optimum concentration was pipetted, and the reaction was incubated at 37°C for 60 minutes followed by washing. Thereafter, 100 µl of a solution of 4 mg/ml of p-nitrophenyl phosphate in 0.05M cabonate buffer was pipetted, the reaction was incubated at 37°C for 60 minutes, and then 100 µl of 1N NaOH was pipetted. This was then added to 2 ml of distilled water and the $OD_{405nm}$ was measured.

Results

The results are given in Table 1.

Table 1

| Specimen | Concentration of EDTA | | | | | |
|----------|------|-------|-------|-------|--------|--------|
|          | 0    | 1 mM  | 3 mM  | 5 mM  | 10 mM  | 20 mM  |
| F        | < 15 ng/ml | < 15 | 268 | 296 | 302 | 309 |
| K        | 30   | 34    | 273   | 262   | 289    | 270    |

As clear from Table 1, it is found that the addition of EDTA has reduced the influences of the inhibitor and the final concentration of 10 mM is satisfactory for measurement as the concentration of EDTA to be added.

Test Example 2

Samples

Samples were prepared by adding respectively $\alpha$-fetoprotein to (1) four fresh serum specimens taken from healthy humans, N, S, T and H, and (2) those obtained by allowing the four specimens to stand at 37°C overnight, in order to give a final concentration of 230 ng/ml, followed by adding EDTA to each specimen until the final concentration of 10 mM to form the test samples, while by without adding EDTA to each specimen to form blank samples.

Method

Measurement was repeated by similar procedures to those described in the section of Method in Test Example 1, except that the anti-m-aldolase antibody was replaced by an anti-$\alpha$-fetoprotein antibody and the alkali phosphatase-labelled anti-m-aldolase antibody was replaced by an alkali phosphatase-labelled $\alpha$-fetoprotein antibody in the section of Method in Test Example 1.

- 8 -

Results

The results are given in Fig. 1. From Fig. 1, it can be found that the addition of EDTA has enhanced the recovery of measured values and that the pretreatment of allowing the sample to stand at 37°C overnight has improved the stability of the percent recovery.

Test Example 3

Samples

The following three kinds of specimens were prepared: (1) a fresh serum taken from a healthy human F, (2) a supernatant obtained by adding kaolin to the same fresh serum to a concentration of 10 mg/ml, allowing it to stand at room temperature for an hour, centrifuging at 3,000 rpm for 20 minutes and obtaining the supernatant, and (3) that obtained by adding EDTA to said supernatant to the final concentration of 10 mM.

$\gamma$-Feto protein to the final concentration of 145 ng/ml was added to each of (1), (2) and (3).

Method

Measurement was carried out by the procedures described in the section of Method in Test Example 2.

Results

The results are given in Table 2.

Table 2

| Specimen | (1) | | (2) | | (3) | |
|---|---|---|---|---|---|---|
| Measured Value | 20 19 20 23 | $\overline{x}$ = 20.5 ng/ml | 64 70 74 70 | $\overline{x}$ = 69.5 ng/ml | 128 120 122 120 | $\overline{x}$ = 122.5 ng/ml |

From Table 2, it can be found that the addition of kaolin has enhanced the recovery of measured values and that the combination of kaolin and EDTA has enhanced the recovery.

Test Example 4

Samples

The following specimens were prepared: (1) fresh serums taken from healthy humans S and N, and (2) supernatants obtained by adding an NHS‑anti‑NHS complex to the same fresh serums respectively to a concentration of 2 mg/ml, allowing each at room temperature for 30 minutes, centrifuging and obtaining each supernatant. And $\alpha$-feto protein was added to each specimen to the final concentration of 330 ng/ml, to prepare each sample.

Method

Measurement was carried out by the procedures described in the section of Method in Test Example 2.

Results

The results are given in Table 3.

Table 3

| | | NHS (Fresh Serum) | | |
|---|---|---|---|---|
| | | S | | N |
| (1) | | 98 ⎫ 89 ⎬ 80 ⎭ | | 45 ⎫ 68 ⎬ 90 ⎭ |
| (2) | | 270 ⎫ 270 ⎬ 270 ⎭ | | 240 ⎫ 255 ⎬ 270 ⎭ |

From Table 3, it can be found that the addition of the NHS - anti-NHS complex has enhanced the recovery of measured values.

Test Example 5

Samples

$\alpha$ -Fetoprotein was added to each fresh serum to a concentration of 230 ng/ml, and thereafter ammonium sulfate was added in the amounts described in the column of Amount Added in Table 4, to prepare each sample.

Method

Measurement was carried out by the procedures described in the section of Method in Test Example 2.

Results

The results are given in Table 4.

Table 4

| Amount Added | Measured Value (ng/ml) | | | | | |
|---|---|---|---|---|---|---|
| None | 18 | 20 | 18 | 17 | $\bar{x} =$ | 18 |
| 12 mM | 21 | 25 | 24 | 30 | | 25 |
| 25 mM | 26 | 29 | 29 | 51 | | 34 |
| 50 mM | 61 | 64 | 60 | 74 | | 65 |
| 100 mM | 170 | 160 | 160 | 160 | | 163 |
| 200 mM | 190 | 200 | 185 | 200 | | 194 |
| 400 mM | 180 | 185 | 182 | 180 | | 182 |

From Table 4, it can be found that the addition of ammonium sulfate has enhanced the recovery of measured values.

Test Example 6

Samples

(1) A fresh serum, (2) that obtained by adding zymosan to the fresh serum to the final concentration of 50 mg/ml, and (3) that obtained by adding anti-Clq to the fresh serum to the final concentration of 0.1 ml/ml were prepared as samples.

Method

m-Aldolase was measured by the procedures described in the section of Method in Test Example 1.

Restuls

The results are given in Table 5

- 12 -

Table 5

| (1) | | (2) | | (3) | |
|---|---|---|---|---|---|
| < 15 ng/ml | }<15 ng/ml | 162 | } 171 ng/ml | 148 | } 149 ng/ml |
| < 15 | | 180 | | 150 | |

From Table 5, it can be found that the addition of zymosan or the addition of anti-Clq has reduced the influences on the measured values by the inhibitor.

Test Example 7

Samples

(1) Three fresh serum specimens taken from healthy humans N, H and T, and (2) three specimens obtained by adding EDTA to the same respective fresh serums to the final concentration of 10 mM were prepared, and then HBs antigen was added to each to the final concentration of 180 ng/ml, to prepare each specimen.

Method

Measurement was carried out by similar procedures to those described in the section of Method in Test Example 1, except that the anti-m-aldolase antibody was replaced by the anti-HBs antibody and the alkali phosphatase-labelled anti-m-aldolase antibody was replaced by an alkali phosphatase-labelled anti-HBs antibody in the section of Method in Test Example 1.

Results

The results are given in Table 6.

Table· 6

| | (1) | (2) |
|---|---|---|
| N | 54 ng/ml | 165 ng/ml |
| | | 165 |
| H | 95 | 160 |
| | | 170 |
| T | 39 | 165 |
| | | 155 |

From Table 6, it can be found that the addition of EDTA has enhanced the recovery of measured values.

The present invention is described in more detail by the following Example.

Example 1

The following reagents (1) - (6) are prepared as described respectively, to provide a reagent kit for measuring the amount of $\alpha$-feto protein in a serum by the solid phase method.

(1) Solid phase substance coated by anti-$\alpha$-fetoprotein antibody

0.05 M Tris hydrochloric acid buffer (pH 8.0) containing 30 $\mu$g/ml of anti-$\alpha$-feto protein antibody is added to a measuring cup, allowed to stand at 4°C overnight, and the solution is removed from the cup, which is then washed with water.

(2) Enzyme-labelled antibody

3 mg of an alkali phosphatase is added to 0.5 ml of 0.05 M tris hydrochloric acid buffer (pH 8.0) containing 3 mg of purified anti-human α-feto protein antibody, to which is added an aqueous glutaraldehyde solution, and the solution is allowed to stand at room temperature for 3 hours, dialyzed against 0.05 M tris-hydrochloric acid buffer (pH 8.0) overnight and lyophilyzed.

(3)  Substrate and Solution for dissolving the substrate p-Nitrophenyl phosphate and 0.05 M carbonate buffer

(4)  Reaction terminating solution 1N NaOH

(5)  standard human α-fetoprotein

(6)  EDTA (adjusted to the final concentration of 10 mM when adding to the specimen).

## CLAIMS

1. A method for measuring the amount of an antigen or antibody in a serum by the solid phase method using an enzyme-labelled antibody, characterized by adding before the measurement one reagent or a combination of two reagents selected from the group consisting of EDTA, kaolin, an antigen-antibody complex, an ammonium salt, zymosan and anti-Clq to the serum or the antigen-antibody reaction system.

2. The method for measuring according to Claim 1 in which the antigen-antibody complex to be added is an immune complex obtained in the NHS - anti-NHS reaction.

3. The method for measuring according to Claim 1 or 2 in which the antigen is $\alpha$-fetoprotein, m-aldolase or HBs antigen.

4. A reagent kit for measuring the amount of an antigen or antibody in a serum by the solid phase method using an enzyme-labelled antibody, which comprises incorporating one reagent or a combination of two reagents selected from the group consisting of EDTA, kaolin, an antigen-antibody complex, an ammonium salt, zymosan and anti-Clq.

5. The reagent kit for measuring according to Claim 4 in which the antigen-antibody complex to be added is an immune complex obtained in the NHS - anti-NHS reaction.

6. The reagent kit for measuring according to Claim 4 or 5 in which the antigen is $\alpha$-fetoprotein, m-aldolase or HBs antigen.

Fig. 1

0068344

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| Y | US-A-4 056 608 (E.ULLMAN et al) *The whole document* | 1,4 | G 01 N 33/54 |
| P,Y | EP-A-0 038 181 (TECHNICON INSTRUMENTS INCORPORATED) *Abstract; page 3, line 30 to page 7, line 20; claims 1-3* | 1,4 | |
| Y | US-A-3 541 202 (H.MEYER et al.) *Example 2* | 1,4 | |
| A | GB-A-2 016 687 (ABBOT LABORATORIES ILLINOIS, USA) *Example 5; claims 1-14* | 3,6 | |
| A | GB-A-2 062 226 (EISAI CO LTD) *The whole document* | 3,6 | **TECHNICAL FIELDS SEARCHED (Int. Cl. ³)** |
| A | FR-A-2 435 715 (SANYO CHEMICAL INDUSTRIES LTD) *Example 7* | 3,6 | G 01 N |

The present search report has been drawn up for all claims

| Place of search | Date of completion of the search | Examiner |
|---|---|---|
| THE HAGUE | 17-09-1982 | OSBORNE H.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82